# EUROPEAN PATENT APPLICATION

(11) **EP 2 039 766 A1**
(43) Date of publication of application: **25.03.2009**
(21) Application number: 07767047.9
(22) Date of filing: 05.06.2007
(51) Int. Cl.: C12N 15/09, C07K 16/28, C12N 5/10, C12N 7/00, A61K 39/00, A61P 35/00

(54) **IMMUNOCOMPETENT CELL HAVING ANTI-CD38 ANTIBODY ON ITS CELL SURFACE**

(30) Priority: 05.06.2006 JP 2006156595
(71) Applicant: Hiroshima University, Higashi-Hiroshima-shi Hiroshima 739-8511 (JP)
(72) Inventor: MIHARA, Keichiro, Hiroshima-shi, Hiroshima 7348553 (JP)
(74) Representative: Cabinet Plasseraud
(86) International application number: PCT/JP2007/061386
(87) International publication number: WO 2007/142241

(57) **Abstract**

An immunocompetent cell expressing an anti-CD38 antibody on its surface through genetic introduction of a DNA coding for part of the anti-CD38 antibody; and a method of producing an immunocompetent cell expressing the antibody on its cell surface, which includes amplifying a cDNA using the mRNA coding for part of the anti-CD38 antibody isolated from a hybridoma, inserting the amplified cDNA into a retroviral vector, transfecting the vector into a packaging cell to produce a packaging cell capable of producing an anti-CD38 antibody expressing-retroviral particle, and infecting a human immunocompetent cell with the retroviral particle released from the packaging cell.

## Description

### TECHNICAL FIELD

The present invention relates to a genetically engineered immunocompetent cell expressing an anti-CD38 antibody and having the antibody on its cell surface. In particular, the present invention relates to a cytotoxic human T cell expressing an anti-CD38 antibody and having the antibody on its cell surface.

### BACKGROUND ART

A CD38 (Cluster of Differentiation 38) antigen is a transmembrane glycoprotein having a molecular weight of 45 kDa and is used as a surface marker for plasma cells, PreB cells, or the like.
The CD38 antigen is involved in cellular differentiation, activation, control of immunological responses, apoptosis and the like. For example, it is known that, in T cells, B cells, and the like, the antigen is expressed in the early stage of generation of the cells and disappears with the maturing of those cells, but the antigen is expressed again when the cells are activated.
It is known that the CD38 antigen which is also expressed in normal cells as described above is expressed on the surfaces of myeloma (such as multiple myeloma) cells, malignant lymphoma cells, and leukemia cells or the like at a level higher than that of the normal cells (see, for example, Non-Patent Document 1).

From the viewpoint of developing a treatment technique for multiple myeloma or the like as described above, a humanized anti-CD38 antibody obtained by cloning of the gene of an antibody against the CD38 antigen, so-called anti-CD38 antibody, has been reported. This report has been made on the phagocytic capacity of monocytes when the antibody is placed *in vitro* in an incubator (Non-Patent Document 2).
However, there is no report that an anti-CD38 antibody has expressed on the surface of an immunocompetent cell. Also, there is no report that investigation is made of the effect of the anti-CD38-expressing immunocompetent cell on myeloma cells, malignant lymphoma cells, or leukemia cells capable of expressing the antigen (CD38) at a high level.

Other and further features and advantages of the present invention will appear more fully from the following description, appropriately referring to the accompanying drawings.

[Non-Patent Document 1] Cytometry, 46: 23-27, 2001
[Non-Patent Document 2] The Journal of Immunology, J. H. Ellis et al., 1995, 155: 925-937

### BRIEF DESCRIPTION OF DRAWINGS

[Fig. 1] Fig. 1 is a schematic view illustrating the sequence of a DNA construct having an anti-CD38 chimeric antibody gene.
[Fig. 2] Fig. 2 is an electrophoresis image, by which it has been confirmed that an anti-CD38 antibody expression retroviral vector includes L- and H-chain genes.
[Fig. 3] Fig. 3 is a two-dimensional plot showing the expression pattern of EGFP and anti-mouse IgG antibody-perCP measured using FACSCalibur (trade name, manufactured by Becton, Dickinson and Company).
[Fig. 4] Fig. 4 is a graph showing the results of a test for evaluation of the function of a human T cell strain having an anti-CD38 antibody on its cell surface.
[Fig. 5] Fig. 5 is a schematic view illustrating suppression of apoptosis by an anti-CD38 antibody.
[Fig. 6] Fig. 6 is a graph showing the results of a test of self-apoptosis suppression by an anti-CD38 antibody (THB7).
[Fig. 7] Fig. 7 is a picture showing the results of a test for evaluation of anti-tumor effect of cells expressing an anti-CD38 antibody.

### DISCLOSURE OF INVENTION

A task of the present invention is to provide a genetically engineered immunocompetent cell expressing an anti-CD38 antibody on its cell surface, which has specific cytotoxicity against myeloma cells, malignant lymphoma cells, leukemia cells, or the like. A further task of the present invention is to provide a cell capable of producing retroviral particles which may infect any immunocompetent cells such as T cells, natural killer cells, macrophages, or polymorphonuclear leukocytes.

The inventor of the present invention has made extensive studies to do the above-mentioned tasks. As a result, it is found that an immunocompetent cell having an anti-CD38 antibody expressed on its cell surface can be produced by a genetic engineering method involving: integrating a cloned anti-CD38 antibody gene into a retroviral vector; transfecting a packaging cell for retrovirus with the resultant retroviral vector to prepare a retroviral particle-producing cell capable of expressing the anti-CD38 antibody; and infecting T cells or the like with the retroviral particles produced by using the retroviral particle-producing cell. The present invention has been made based on such findings.

According to the present invention, there is provided the following means:
(1) An immunocompetent cell expressing an anti-CD38 antibody on its surface through genetic introduction of a DNA coding for part of the anti-CD38 antibody;
(2) The immunocompetent cell according to the above item (1), wherein the anti-CD38 antibody is an antibody having the following amino acid sequence (a) or (b) in its variable region:
   (a) An amino acid sequence set forth in SEQ ID NO: 1; and
   (b) An amino acid sequence, in which one or more amino acids are being deleted, substituted or added in the amino acid sequence set forth in SEQ ID NO: 1;
(3) The immunocompetent cell according to the above item (1) or (2), wherein the anti-CD38 antibody is an antibody that has the following amino acid sequence (c) or (d) in its heavy chain variable region:
   (c) An amino acid sequence set forth in SEQ ID NO: 2; and
   (d) An amino acid sequence, in which one or more amino acids are being deleted, substituted or added in the amino acid sequence set forth in SEQ ID NO: 2;
(4) The immunocompetent cell according to the item above item (1), wherein the DNA coding for part of the anti-CD38 antibody has a DNA coding the following amino acid sequence (b) and a DNA coding the following amino acid sequence (d) introduced thereinto:
   (b) An amino acid sequence, in which one or more amino acids are being deleted, substituted or added in the amino acid sequence set forth in SEQ ID NO: 1; and
   (d) An amino acid sequence, in which one or more amino acids are being deleted, substituted or added in the amino acid sequence set forth in SEQ ID NO: 2;

(5) The immunocompetent cell according to the above item (1), wherein the anti-CD38 antibody has an amino acid sequence set forth in SEQ ID NO: 1 in part of its light chain and an amino acid sequence set forth in SEQ ID NO: 2 in part of its heavy chain;
(6) The immunocompetent cell according to any one of the above items (1) to
(5), wherein the immunocompetent cell is a T cell, natural killer cell, macrophage or polymorphonuclear leukocyte;

(7) A protein of the following (a1), (b1), (c1) or (d1):
   (a1) A protein having an amino acid sequence set forth in SEQ ID NO: 1;
   (b1) A protein having an amino acid sequence, in which one or more amino acids are being deleted, substituted or added in the amino acid sequence set forth in SEQ ID NO: 1;
   (c1) A protein having an amino acid sequence set forth in SEQ ID NO: 2; and
   (d1) A protein having an amino acid sequence, in which one or more amino acids are being deleted, substituted or added in the amino acid sequence set forth in SEQ ID NO: 2;
(8) A DNA coding the following amino acid sequence (a), (b), (c) or (d):
   (a) An amino acid sequence set forth in SEQ ID NO: 1;
   (b) An amino acid sequence, in which one or more amino acids are being deleted, substituted or added in the amino acid sequence set forth in SEQ ID NO: 1;
   (c) An amino acid sequence set forth in SEQ ID NO: 2; and
   (d) An amino acid sequence, in which one or more amino acids are being deleted, substituted or added in the amino acid sequence set forth in SEQ ID NO: 2;
(9) A recombinant retrovirus having a DNA coding the following amino acid sequence (a), (b), (c) or (d):
   (a) An amino acid sequence set forth in SEQ ID NO: 1;
   (b) An amino acid sequence, in which one or more amino acids are being deleted, substituted or added in the amino acid sequence set forth in SEQ ID NO: 1;
   (c) An amino acid sequence set forth in SEQ ID NO: 2; and
   (d) An amino acid sequence, in which one or more amino acids are being deleted, substituted or added in the amino acid sequence set forth in SEQ ID NO: 2;
(10) A packaging cell capable of producing particles of the recombinant retrovirus according to the above item (9);

(11) An anti-CD38 antibody having the following amino acid sequence (a) or (b) in its light chain variable region and having the following amino acid sequence (c) or (d) in its heavy chain variable region:
   (a) An amino acid sequence set forth in SEQ ID NO: 1;
   (b) An amino acid sequence, in which one or more amino acids are being deleted, substituted or added in the amino acid sequence set forth in SEQ ID NO: 1;
   (c) An amino acid sequence set forth in SEQ ID NO: 2; and
   (d) An amino acid sequence, in which one or more amino acids are being deleted, substituted or added in the amino acid sequence set forth in SEQ ID NO: 2;

(12) A method of producing an immunocompetent cell expressing an anti-CD38 antibody on its cell surface, comprising the steps of:
   amplifying a cDNA of the anti-CD38 antibody using the mRNA coding part of the anti-CD38 antibody isolated from a hybridoma capable of producing the anti-CD38 antibody;
   inserting the amplified cDNA into a retroviral vector;
   transfecting the gene-inserted vector into a packaging cell to produce a packaging cell capable of producing an anti-CD38 antibody-expressing retroviral particle; and
   infecting a human immunocompetent cell with the retroviral particle released from the packaging cell;
(13) The method of producing an immunocompetent cell according to the above item (12), wherein the anti-CD38 antibody is the antibody according to the above item (11);
(14) The method of producing an immunocompetent cell according to the above item (12) or (13), wherein the cDNA is the DNA according to the above item (8);
(15) The method of producing an immunocompetent cell according to any one of the above items (12) to (14), wherein the packaging cell is the cell according to the above item (10);
(18) The method of producing an immunocompetent cell according to any one of the above items (12) to (15), wherein the immunocompetent cell is the cell according to any one of the above items (1) to (6); and
(17) The method of producing an immunocompetent cell according to any one of the above items (12) to (16), wherein an anti-CD38 antibody is allowed to coexist when the anti-CD38 antibody is expressed from a human immunocompetent cell infected with the retroviral particle.

### BEST MODE FOR CARRYING OUT THE PRESENT INVENTION

### [Summary of the Invention]

A conventional anticancer chemotherapeutic agent has a problem in that an anticancer agent has an effect to attack not only cancer cells but also normal cells or normal tissues and has an essentially insoluble problem that a strong anticancer effect leads to strong side effects. The present invention provides a useful method for solving such problems.
The present invention is based on properties of an immunocompetent cell having cytotoxic activity against foreign matters from the outside (such as bacteria and viruses) or against abnormal cells (such as cancer cells) spontaneously generated in the body. Therefore, among immunocompetent cells, T cells, NK cells, macrophages, and polymorphonuclear leukocytes, which have cytotoxic activity, are preferably used. However, the immunocompetent cell of the present invention does not include B lymphocytes which usually produce humoral antibodies in a living body. The immunocompetent cell of the present invention is obtained by expressing an antibody against a CD38 antigen (anti-CD38 antibody) of another lymphocyte at a high density on the surface of the above-mentioned immunocompetent cell having cytotoxic activity. Such a cell has strong affinity for cancer cells (such as malignant lymphoma or myeloma) having the anti-CD38 antigen on its surface. Therefore, when a cell having the antibody expressed is returned to the body of a patient, the antibody easily binds to the cancer cell. Then, the cancer cell is killed by the cytotoxic activity intrinsically possessed by the immunocompetent cell, thereby treating the cancer.

A treatment method involving systemic administration of an antibody or topical administration of an antibody in a considerable amount has been performed conventionally, and even now, such a treatment method is mainly employed as an antibody therapy. However, such a treatment method has essential problems such as rejection caused by systemic administration or topical administration of antibodies produced in different individuals and lowering of a therapeutic effect due to a change in antigens on the surface of a cancer cell or lowering of affinity.
The present invention relates not to a method of using an antibody for systemic administration to a patient, but to a technique of returning an immunocompetent cell collected from a patient, genetically engineering the cell so that the antibody is expressed on the cell surface, to the same patient. As a result, it is possible to provide an effective technique for solving problems such as side-effects caused by conventional systemic administration of an antibody and lowering of an anticancer effect due to a change in cancer antigens.

### [Application]

The immunocompetent cell having the anti-CD38 antibody on its surface of the present invention targets abnormal cells (such as malignant lymphoma) which express a CD38 antigen at a high level compared with normal cells. Specific examples of the target abnormal cells include myeloma cells, malignant lymphoma cells, leukemia cells expressing the CD38 antigen at a high level, and plasma cells in autoimmune disease.
Hereinafter, the immunocompetent cell of the present invention will be described in detail. The immunocompetent cell of the present invention has the anti-CD38 antibody expressed on its surface by a genetic manipulation technique.

### [Immunocompetent cell]

In the case of a treatment for human cancer, the cells that may be used as the immunocompetent cells of the present invention must be human cells, which is the same species, for the purpose of preventing rejection caused by transplantation, and the immunocompetent cells which are the same species and are derived from the same individual must be used. However, the immunocompetent cells of the present invention do not include B lymphocytes merely producing humoral antibodies against the CD38 antigen.
Also in the case where such immunocompetent cells are applied to animals, immunocompetent cells derived from the same individual of the same species (as an individual to be treated) are preferably used. Therefore, in the case where the present invention is used in human clinical practice, immunocompetent cells collected from a patient to be treated with malignant lymphoma, myeloma, or the like are preferably used. Blood may be collected by any method, and peripheral blood is preferably used from the viewpoint of convenience and easiness of the collection. Blood may be collected in a small amount, and the amount ranging from 50 ml to 400 ml is sufficient.

The immunocompetent cells to be used in the present invention preferably have cytotoxicity (cytotoxic activity) against abnormal cells such as cancer cells. Examples of the cells include lymphocytes, mononuclear cells, and polymorphonuclear leukocytes. Of those immunocompetent cells, lymphocytes are preferable, and of those lymphocytes, T cells are preferable. Moreover, of those T cells, killer T cells or NK cells (natural killer cells) having cytotoxicity are more preferable. Of those mononuclear cells, cells having phagocytic activity such as macrophages and phagocytes are preferable. Of those polymorphonuclear leukocytes (PMN), any polymorphonuclear leukocytes having phagocytic activity may be used as the immunocompetent cells of the present invention.

The immunocompetent cells to be used in the present invention are preferably obtained by selecting and separating immunocompetent cell species (such as T cells or macrophages) from blood collected from a patient to be treated. It is not always necessary to exactly separate the cell species of the immunocompetent cells to be used into small subsets. Thus, the immunocompetent cells of the present invention to be used may include: cells mainly containing a T cell group, which are used as lymphocytes; cells having phagocytic activity (such as macrophages) at a high level, which are used as mononuclear cells; and a cell group containing polymorphonuclear leukocytes (PMN) having phagocytic activity at a high level. In order to merely achieve therapeutic effects, it is not necessary to exactly identify and separate/select the cell species. However, if immunocompetent cells to be used must be exactly selected depending on research purposes and therapeutic purposes, a cell group including immunocompetent cells suitable for the purposes may be prepared.

### [Separation of immunocompetent cell]

In the case where immunocompetent cells are separated from blood taken, for example, by blood collection, from a patient with malignant lymphoma or a healthy individual (control) by specific gravity separation or the like, the immunocompetent cells may be obtained by centrifuging the blood using Ficoll or the like and collecting a lymphocyte group-rich layer in the middle-layers. In the case of using lymphocytes, the resultant lymphocyte-rich cell group may be cultured in the presence of IL-2 or the like at a predetermined concentration under predetermined conditions by an ordinary method to obtain a cell population including only T cells or a T-cell-rich cell group. In addition, mononuclear cells such as macrophages may be selected based on adhesion properties to a culture container. Polymorphonuclear cells may be separated from macrophages in a cell group obtained from peripheral blood through selection by the specific gravity method and based on a difference in the degrees of adhesion properties.

### [Anti-CD38 antibody]

The anti-CD38 antibody according to the present invention is a protein having the following amino acid sequence (a) or (b).
(a) An amino acid sequence set forth in SEQ ID NO: 1
(b) An amino acid sequence, in which one or more amino acids are being deleted, substituted or added in the amino acid sequence set forth in SEQ ID NO: 1
The amino acid sequence (a) or (b) constitutes part of the light chain variable region of the CD38 IgG antibody. Also, the amino acid sequence (b) preferably has biological functions as part of the light chain variable region of the anti-CD38 antibody.

The anti-CD38 antibody of the present invention is a protein having the following amino acid sequence (c) or (d).
(c) An amino acid sequence set forth in SEQ ID NO: 2
(d) An amino acid sequence, in which one or more amino acids are being deleted, substituted or added in the amino acid sequence set forth in SEQ ID NO: 2
In the case where the anti-CD38 antibody of the present invention has the amino acid sequence (d), the amino acid sequence includes only an amino acid sequence capable of providing an antibody having affinity for the CD38 antigen and does not include an amino acid sequence (d) capable of providing an antibody having no affinity for the CD38 antigen.

The amino acid sequences (c) and (d) constitute parts of the heavy chain variable region of the anti-CD38 antibody. Therefore, a protein having the sequence (c) or (d) preferably has biological and immunological functions of the heavy chain variable region.
The anti-CD38 antibody of the present invention particularly preferably has the amino acid sequence set forth in SEQ ID NO: 1 in the light chain and the amino acid sequence set forth in SEQ ID NO: 2 in the heavy chain. Moreover, the anti-CD38 antibody of the present invention is preferably an antibody protein having the amino acid sequence (a) or (b) in the light chain and having the amino acid sequence represented by (c) or (d) in the heavy chain. Accordingly, one antibody protein may include a protein having (a) and (b), a protein having (a) and (c), a protein having (b) and (c), and a protein having (b) and (d) in combination. Of those, the protein having (a) and (c) is most preferable, and the protein having (a) and (d) is more preferable.

The anti-CD38 antibody of the present invention has a structure of the above-mentioned sequences and has functions of an antibody against the CD38 antigen, which is one of lymphocyte surface antigens. Therefore, the antibody has strong affinity for the CD38 antigen.
In addition, a preferable aspect is as follows: the anti-CD38 antibody of the present invention is an antibody in which an antibody protein is expressed on the surface of an immunocompetent cell by a gene DNA introduced by a viral vector by using the mechanism of the cell in which the gene DNA is introduced.

In the case where the present invention is applied to treatments of human malignant lymphoma, myeloma or the like, the CD38 antigen to be used is preferably a CD38 antigen of human lymphocytes or the like. In the present invention relating to a treatment based on an immunological mechanism, an antibody or a serum containing an antibody is not systemically administered, but a special aspect in which the treatment is performed using part of a human antibody is employed. Therefore, it is not necessary to consider differences between antigens/antibodies of individuals as an issue.

The term "anti-CD38 antibody" as used herein refers to an antibody having an amino acid sequence of SEQ ID NO: 1 or an amino acid sequence of SEQ ID NO: 1 with substitution, deletion, or addition of one or several amino acids in its light chain variable region and an amino acid sequence of SEQ ID NO: 2 or an amino acid sequence of SEQ ID NO: 2 with substitution, deletion, or addition of one or several amino acids in its heavy chain variable region. The antibody preferably has the amino acid sequence of SEQ ID NO: 1 in its light chain variable region and an amino acid sequence of SEQ ID NO: 2 in its heavy chain variable region. Further, the amino acid sequence of SEQ ID NO: 1 or 2 with substitution of one or several amino acids is limited to an amino acid sequence with substitution, deletion, or addition of an antibody having affinity for a CD38 antigen.

### [Gene coding anti-CD38 antibody]

The anti-CD38 antibody to be used in the present invention is a protein in the variable region of an IgG antibody or an antibody protein having the IgG variable region. The IgG variable region includes a light chain and a heavy chain. Therefore, the anti-CD38 antibody of the present invention also includes a variable light chain region and a variable heavy chain region.

The DNA of the light chain variable region in a gene coding the anti-CD38 antibody is a DNA having a DNA sequence coding the following amino acid sequence (a) or (b) or a DNA having the DNA sequence as part.
(a) An amino acid sequence set forth in SEQ ID NO: 1
(b) An amino acid sequence, in which one or more amino acids are being deleted, substituted or added in the amino acid sequence set forth in SEQ ID NO: 1
In the case where the anti-CD38 antibody of the present invention has the amino acid sequence (b), the amino acid sequence includes only an amino acid sequence capable of providing an antibody having affinity for the CD38 antigen and does not include the amino acid sequence (b) capable of providing an antibody having no affinity for the CD38 antigen.

The DNA sequence of the present invention may be a DNA that hybridizes with a DNA coding the amino acid sequence (a) or (b) under stringent conditions.

The nucleotide sequence of the heavy chain variable region in a gene coding the anti-CD38 antibody of the present invention is a nucleotide sequence having a DNA nucleotide sequence coding the following amino acid sequence (c) or (d) or a nucleotide sequence having the nucleotide sequence as part.
(c) An amino acid sequence set forth in SEQ ID NO: 2
(d) An amino acid sequence, in which one or more amino acids are being deleted, substituted or added in the amino acid sequence set forth in SEQ ID NO: 2
In the case where the gene coding the anti-CD38 antibody of the present invention has the amino acid sequence (d), the amino acid sequence includes only an amino acid sequence (d) capable of providing an antibody having affinity for the CD38 antigen and does not include an amino acid sequence (d) capable of providing an antibody having no affinity for the CD38 antigen.

The above-mentioned DNA nucleotide sequence may be a nucleotide sequence that hybridizes with a DNA nucleotide sequence coding for the amino acid sequence (c) or (d) under stringent conditions.

The gene coding the anti-CD38 antibody of the present invention preferably has a DNA nucleotide sequence coding an antibody protein having either the amino acid sequence (a) or (b) and either the amino acid sequence (c) or (d) on the antibody including the same protein molecule.

The anti-CD38 antibody of the present invention preferably has the amino acid sequence set forth in SEQ ID NO: 1 in its light chain and the amino acid sequence set forth in SEQ ID NO: 2 in its heavy chain. Therefore, the DNA coding the anti-CD38 antibody having the amino acid sequence set forth in SEQ ID NO: 1 in the light chain and the amino acid sequence set forth in SEQ ID NO: 2 in the heavy chain is included in a gene coding the anti-CD38 antibody of the present invention (referred to as "DNA construct 1") and is a DNA in a more preferable aspect.
In the case where a DNA (sequence) coding the amino acid sequence of SEQ ID NO: 1 or 2 with substitution, deletion or addition of one or several amino acids is introduced into an immunocompetent cell, the phrase "DNA is introduced into an immunocompetent cell" means that the DNA (sequence) is integrated into chromosomal DNA of the immunocompetent cell, and the protein is expressed.

The nucleotide sequence coding a protein having the amino acid sequence (a) set forth in SEQ ID NO: 1 or the amino acid sequence (b) set forth in SEQ ID NO: 2 is a nucleotide sequence determined as follows: the gene of the anti-CD38 antibody is cloned by the method described below; and the nucleotide sequence is determined based on the cloned sequence of the gene.

### [Immunocompetent cell expressing anti-CD38 antibody on its cell surface]

Next, a method of producing an immunocompetent cell expressing an anti-CD38 antibody on its surface will be described.
The immunocompetent cell having the anti-CD38 antibody on its surface of the present invention is produced by (1) a step of producing a hybridoma, (2) a step of isolating mRNA from the hybridoma, amplifying cDNA, and cloning the cDNA of the anti-CD38 antibody, (3) a step of amplifying part of the antibody cDNA to produce a gene having the antibody cDNA such as "DNA construct 1", (4) a step of constructing a retroviral vector capable of expressing the anti-CD38 antibody, (5) a step of transfecting a packaging cell with the viral vector to produce a packaging cell, (6) a step of infecting immunocompetent cells prepared from blood collected from a patient with retroviral particles (virions) released from the resultant packaging cell, and (7) a step of selecting an immunocompetent cell expressing the antibody at an appropriate level among immunocompetent cells infected with the particles and expressing the antibody.

The present invention can be carried out, in the stated order, by the steps of producing and selecting the immunocompetent cell having the anti-CD38 antibody on its cell surface of the present invention.

### (1) [Step of producing hybridoma]

First, it is necessary that a hybridoma capable of producing an antibody against the CD38 antigen be produced. A preferable hybridoma capable of producing the anti-CD38 antibody can be obtained by an ordinary method involving: administering the antigen to an animal; enhancing production of the antibody with a booster; fusing spleen cells of an animal showing a high antibody titer with myeloma cells to produce hybridomas; and screening the resultant hybridomas based on the production of the anti-CD38 antibody as an indicator. To facilitate the process, a hybridoma THB7 commercially available from ATCC may also be used.

Examples of such hybridoma cells include THB7 cell (ATCC (American Type Culture Collection) HB-136), AT 13/5 cell, and HIT2 cell. The THB7 cell is preferably used.

### (2) [Step of cloning cDNA]

Total mRNA can be extracted from the hybridoma cell by an ordinary method such as a method using Trizol reagent (trade name, manufactured by GIBCO-BRL). The mRNA isolated from the hybridoma is converted into cDNA and then amplified as a DNA by a technique such as PCR. Among PCR methods, RT-PCR is preferably employed to convert the RNA to DNA. Primers to be used for cloning of DNAs coding the amino acid sequences of SEQ ID NOS: 1 and 2 can be prepared according to, for example, the method by J. H. Ellis et al. (The Journal of Immunology, 1995, 155: 925-937). Specifically, (i) for the heavy chain variable region of the antibody (SEQ ID NO: 2), primers A_{H} to H_{H} described in the document by J. H. Ellis et al. (The Journal of Immunology, 1995, 155: 925-937) may be used as primers. Meanwhile, (ii) for the light chain variable region of the antibody (SEQ ID NO: 1), primers A_{L} to H_{L} described in the document may be used as primers.

By amplifying an ordinary method such as PCR, cDNA coding for H-chain variable region (SEQ ID NO: 2) and the L-chain variable region (SEQ ID NO: 1) can be obtained. The resultant cDNA is cloned into an appropriate vector (such as pUC18) to determine the nucleotide sequence, and the method of determining the nucleotide sequence is not limited, and any method such as the dideoxy terminator method can be used for determination.

### (3) [Preparation of DNA construct 1 having anti-CD38 antibody gene (cDNA)]

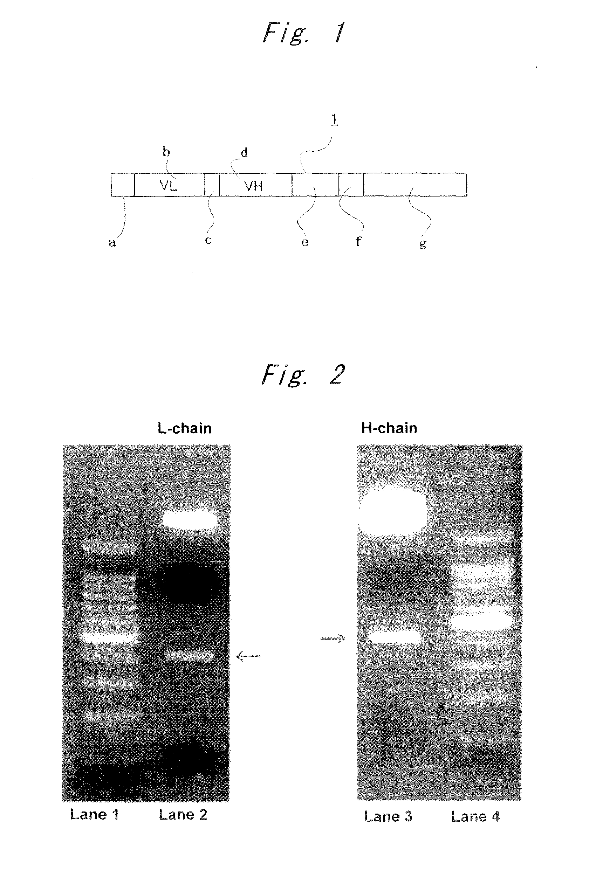

The DNA construct 1 having an anti-CD38 antibody gene (Fig. 1) is prepared by, for example, sequentially ligating (a) a nucleotide sequence coding a signal peptide of CD8α, (b) a nucleotide sequence coding the L-chain variable region (SEQ ID NO: 1), (c) a linker nucleotide sequence, (d) a nucleotide sequence coding the H-chain variable region (SEQ ID NO: 2), (e) a nucleotide sequence coding hinge and transmembrane domains of CD8α, (f) a nucleotide sequence coding a 4-1 BB intracellular domain, and (g) a nucleotide sequence coding a CD3ζ chain.
A DNA construct for expressing an anti-CD38 chimeric antibody can be prepared from the resultant cDNA coding the H-chain variable region (SEQ ID NO: 2) and the L-chain variable region (SEQ ID NO: 1) according to, for example, a method by Imai et al. (C. Imai, K. Mihara et al, Leukemia, 2004, 18:676-684).

Specifically, the DNA construct 1 for expressing an anti-CD38 chimeric antibody can be prepared by introducing (b) a sequence coding the L-chain variable region (SEQ ID NO: 1) and (d) a sequence coding the H-chain variable region (SEQ ID NO: 2) into an expression cassette obtained by sequentially ligating (a) a sequence coding a signal peptide of CD8α, (c) a linker sequence, and (e) a sequence coding hinge and transmembrane domains of CD8α (Fig. 1).

In the DNA construct 1, (b) the sequence coding the L-chain variable region (SEQ ID NO: 1) may be introduced between (e) the sequence coding a signal peptide of CD8α and (c) the linker sequence by the SOC method, while (d) the sequence coding the H-chain variable region (SEQ ID NO: 2) may be introduced between (c) the linker sequence and (e) the sequence coding hinge and transmembrane domains of CD8α by the SOC method.

### (4) [Construction of anti-CD38 antibody expression retroviral vector]

Next, the "construction of anti-CD38 antibody expression retroviral vector" will be described. The DNA construct 1 having the anti-CD38 antibody gene, produced as above, may be integrated into a viral vector to construct a viral vector for expressing the anti-CD38 antibody.
The viral vector to be used in the present invention is not particularly limited as long as the generated viral particles (virions) can infect immunocompetent cells such as human T cells, and the cells can express the anti-CD38 antibody. The vector is preferably a retroviral vector from the viewpoint in which a gene to be introduced (having the anti-CD38 antibody gene) is integrated into the chromosome of a host cell at high efficiency and a high level of the expression of the gene can be maintained. More specifically, the vector is more preferably a recombinant retroviral expression vector having a DNA coding the amino acid sequences set forth in SEQ ID NOS: 1 and 2.

The term "retroviral vector" as used herein refers to a so-called oncoretroviral vector (hereinafter, simply referred to as "retroviral vector"). The above-mentioned retroviral vector may be a lentiviral vector or the like.
In general, the retroviral vector has a structure in which there are a *gag*/*pol* gene coding a precursor protein that becomes a viral structural protein (such as a protease, reverse transcriptase, or integrase) and an *env* gene coding an envelope glycoprotein, and the both ends are sandwiched by LTRs (long terminal repeats) including elements such as an enhancer, promoter, and polyadenylation signal.

Examples of the retroviral vector to be used in the present invention include MSCV (murine stem cell virus) and lentivirus. In particular, the MSCV is more preferable from the viewpoint of preventing suppression of virus expression (silencing) in transgenic cells by methylation of LTR and from a safety standpoint.

In the present invention, MSCV Retroviral Expression System (trade name, manufactured by BD Biosciences Clontech) may also be used as a product similar to the retroviral vector. In the case where MSCV is used as the retroviral vector, the DNA construct having the anti-CD38 antibody gene is preferably introduced by an ordinary method into the restriction enzyme *Eco*RI-restriction enzyme *Xho*I site in a multicloning site in MSCV in a direction suitable for expression.

The retroviral vector to be used in the present invention preferably has an IRES (internal ribosome entry site) sequence from the viewpoint in which translation of eukaryotic mRNA is based on cap-structure-independent translation. The IRES sequence is a sequence that is present in an mRNA strand and codes a structure which binds directly to a ribosome, and the sequence is used in a mechanism to initiate translation by the direct binding of ribosome, a so-called internal initiation mechanism.

The retroviral vector to be used in the present invention preferably has any selection (marker) gene for the purpose of selecting the anti-CD38 antibody-expressing immunocompetent cell of the present invention or transgenic cell, for example, analyzing the cell by flow cytometry, or facilitating the process of isolation. Examples of the selection gene include EGFP (enhanced green fluorescent protein) and GFP, and the EGFP is preferable.

The retroviral vector before transfection into a packaging cell described below to form anti-CD38 antibody-expressing retroviral virions is preferably a plasmid vector (so-called transfer vector plasmid). In particular, from the viewpoint of forming retroviral virions in combination with a packaging cell for retrovirus, the retroviral vector is more preferably a transfer vector plasmid which does not include a part or whole of the *gag*/*pol* gene and a part or whole of the *env* gene (which have the anti-CD38 antibody gene instead) and forms no retroviral particles (virions) when used singly because the *gag*/*pol* gene and *env* gene are not expressed.

Such a transfer vector plasmid can form a complete retroviral virion including RNA genome having an anti-CD38 antibody gene incorporated thereinto by simultaneously transfecting (co-transfecting) a packaging cell for retrovirus with a helper vector plasmid (having part of *gag*/*pol*) and an envelope vector plasmid (having env). Alternatively, the complete retroviral virion can also be obtained by transfecting a packaging cell having only *gag*/*pol* and *env* with the transfer vector plasmid.

MSCV plasmid including IRES and EGFP, which is preferably used as the retroviral vector plasmid in the present invention, is available from, for example, St. Jude Vector Development and Production Shared Resource (Memphis, TN, USA).

### (5) [Step of producing packaging cell]

A step of producing a packaging cell by transfecting a packaging cell with the viral vector will be described. A cell capable of producing anti-CD38 antibody-expressing retroviral particles (virus virions) can be produced by transfecting a packaging cell with the viral vector having the gene inserted.

A step of infecting an immunocompetent cell prepared from blood collected from a patient with retrovirus virions released from the packaging cell will be described.
In order to form the anti-CD38 antibody-expressing retroviral particles (virions) in a packaging cell, the retroviral vector is preferably incorporated into the packaging cell by an ordinary transfection method such as lipofection.

In the packaging cell, it is necessary to form complete retroviral virions which include RNA genome having the anti-CD38 antibody gene (DNA construct 1), and a packaging cell suitable for retrovirus (packaging cell for retrovirus) is preferably used.
The term "packaging cell for retrovirus" as used herein refers to a packaging cell which has the complete *gag*/*pol* and *env* genes described above but cannot form retroviral virions including RNA genome when used singly. Examples of the packaging cell for retrovirus include 293T cell (Riken Bioresource Center, Accession No. RCB2202).

The cell capable of producing anti-CD38 antibody-expressing retroviral particles of the present invention is produced by introducing the recombinant retroviral expression vector into a packaging cell. Most preferably, the recombinant retroviral expression vector has a DNA coding the amino acid sequences set forth in SEQ ID NOS: 1 and 2, but even if the vector has a DNA coding the amino acid sequences set forth in SEQ ID NOS: 1 and 2 with substitution, deletion, or addition of one or several amino acids, the vector can be used as the vector of the present invention.

The packaging cell produced by the present inventor and the like for the purpose of producing the anti-CD38 antibody-expressing retroviral particles (virions) (CAR38 293T cell) has been deposited at the International Patent Organism Depositary of the National Institute of Advanced Industrial Science and Technology (Central 6, 1-1, Higashi 1-chome, Tsukuba-shi, Ibaraki-ken, 305-8566, Japan) on February 15, 2006 (Accession No.: FERM AP-20800).
The culture conditions for forming the anti-CD38 antibody-expressing retroviral particles are not particularly limited, but the cell is preferably incubated in any medium such as RPMI 1640 containing fetal calf serum at 37°C for 24 to 96 hours.
The resultant anti-CD38 antibody-expressing retroviral particles can be obtained by collecting a medium or a culture solution. In order to identify the cell capable of producing the viral particles, expression of GFP may be detected by flow cytometry to select or detect a target anti-CD38 antibody-expressing cell.

### (6) [Production of immunocompetent cell having anti-CD38 antibody on its cell surface of the present invention]

Hereinafter, a step of infecting a human immunocompetent cell with viral particles released from the packaging cell produced in the above-mentioned step is described. Human T cells can be infected with the viral particles by (A) infecting the immunocompetent cells using a culture supernatant of cells capable of producing the anti-CD38 antibody-expressing retroviral particles or (B) infecting the immunocompetent cells with the viral particles by mixed culture of the cells capable of producing the retroviral particles and the immunocompetent cells. As a result, some of the immunocompetent cells infected with the viral particles have the viral particles temporarily in their cells. However, in some of the immunocompetent cells, RNA of the retrovirus is converted into DNA and incorporated into and fixed in chromosomal DNA of a host. The culture conditions for infecting immunocompetent cells with the retroviral particles are not particularly limited, but the cells are preferably incubated in any medium such as RPMI 1640 containing fetal calf serum at 37°C for 24 to 96 hours.

The human T cell (CAR38-Hut78) infected with the virus, which is obtained by using a culture solution of the packaging cell capable of producing the viral particles (CAR38-293T cell) or obtained by coculture with the packaging cell (CAR38-293T cell), has been deposited at the International Patent Organism Depositary of the National Institute of Advanced Industrial Science and Technology (Central 6, 1-1, Higashi 1-chome, Tsukuba-shi, Ibaraki-ken, 305-8566, Japan) on February 15, 2006 (FERM AP-20801).

Examples of the immunocompetent cells to be infected with the anti-CD38 antibody-expressing retroviral particles include any T cells (T lymphocytes) (such as cytotoxic T cells (killer T cells), helper T cells, and suppressor T cells), natural killer (NK) cells, macrophages, and polymorphonuclear leukocytes (PMN). The immunocompetent cells are preferably the cytotoxic T cells or natural killer (NK) cells, more preferably the cytotoxic T cells, and still more preferably cytotoxic human T cells.

A specific example of the T cells to be infected includes a human T cell-based cell strain (Hut78 cell; Institute of Development, Aging and Cancer, Tohoku University, Accession No. TKG0375). Another specific example of the immunocompetent cells to be infected includes Hut102 cell strain (Institute of Development, Aging and Cancer, Tohoku University, Accession No. TKG0382).

The immunocompetent cell having the CD38 antibody on its cell surface of the present invention, which is obtained by infecting any immunocompetent cell isolated from a patient with a disease caused by CD38 (such as myeloma, malignant lymphoma, or leukemia) with anti-CD38 antigen-expressing retroviral virions, may be returned to the patient for the purpose of treating the disease. This can prevent rejection caused by recognition of the immunocompetent cell having the anti-CD38 antibody on its cell surface of the present invention as "non-self" due to an individual difference.

### (7) [Selection of immunocompetent cell having anti-CD38 antibody on its cell surface]

Selection of the immunocompetent cell having the anti-CD38 antibody on its cell surface of the present invention is described.
In general, even in normal cells (such as T cells and B cells), the CD38 antigen is expressed in an amount smaller than that in the above-mentioned target cells such as malignant lymphoma cells and leukemia cells, and the normal cells have a small amount of the CD38 antigen on their cell surfaces. However, the CD38 expression level in myeloma cells is almost the same as that in plasma cells.

The immunocompetent cells having the anti-CD38 antibody on their cell surfaces, which are produced by infection as described above, may include a cell having the anti-CD38 antibody on its cell surface in a very large amount depending on the degree of the infection or expression. The cells having a very large amount of the anti-CD38 antibody on their cell surfaces exert functions such as cytotoxicity not only against the target cells such as myeloma cells, malignant lymphoma cells, and leukemia cells but also against normal cells having a small amount of the CD38 antigen on their cell surfaces and are not specific to the target malignant cells.

Therefore, the immunocompetent cells having the anti-CD38 antibody on their cell surfaces (in particular, human T cells having the anti-CD38 antibody on their cell surfaces), which are produced by infection as described above, are preferably obtained by isolating and selecting only cells which have no or little cytotoxicity against normal cells (such as T cells or B cells) but have sufficient cytotoxicity against above-mentioned target cells such as myeloma cells, malignant lymphoma cells, and leukemia cells.

The vectors used include GFP (green fluorescence protein) or EGFP (enhanced fluorescence protein) inserted thereinto. The GFP or EGFP is positively correlated with expression of the anti-CD38 antibody, and an immunocompetent cell having the anti-CD38 antibody expressed arbitrarily can be easily selected and separated using a flow cytometer.

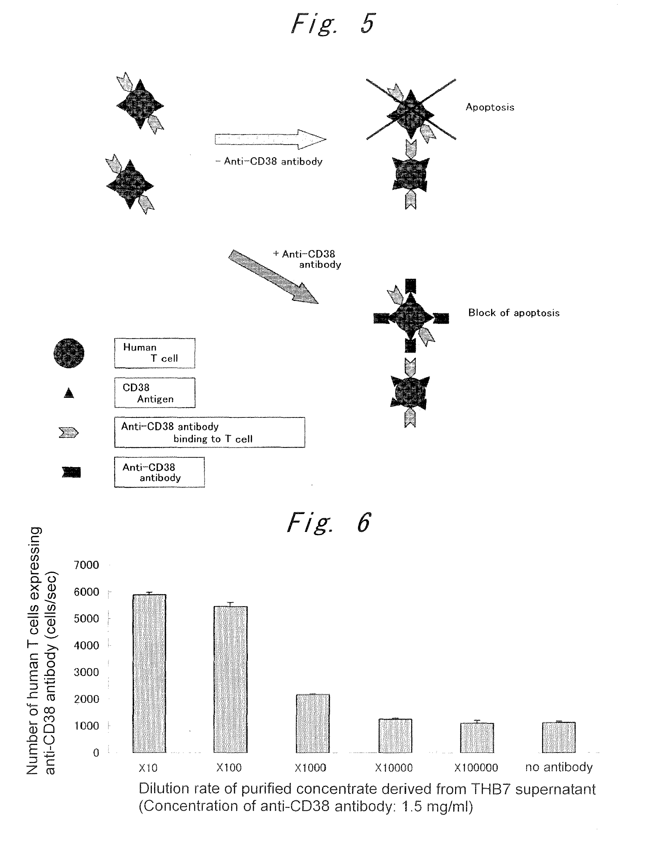

In the method of producing the immunocompetent cell of the present invention, an anti-CD38 antibody is preferably added in advance before expression of the anti-CD38 antibody from a human immunocompetent cell infected with retroviral particles.
As shown in Fig. 5, the CD38 antigen is expressed on a human T cell or the like, and even if an immunocompetent cell obtained by expressing the anti-CD38 antibody in a human T cell is produced by the method of producing the immunocompetent cell of the present invention, the immunocompetent cells interfere each other to cause apoptosis.
On the other hand, when an anti-CD38 antibody is added in advance before expression of the anti-CD38 antibody by a human immunocompetent cell infected with retroviral particles, the added anti-CD38 antibody can recognize the CD38 antigen expressed on the surface of the immunocompetent cell having the expressed anti-CD38 antibody, and the immunocompetent cells interfere each other to prevent apoptosis.

The anti-CD38 antibody to be added is not particularly limited as long as the antibody can recognize the CD38 antigen expressed on the surface of the immunocompetent cell having the expressed anti-CD38 antibody and can compete with the expressed anti-CD38 antibody. Specifically, an anti-CD38 antibody derived from THB7 is preferable.
The coexistence concentration of the anti-CD38 antibody is not particularly limited, but the concentration is preferably about 0.015 mg/ml or more, more preferably about 0.15 mg/ml or more with respect to 1 × 10⁵ to 1 × 10⁶ human T cells.

### [Function of immunocompetent cell]

Hereinafter, the "function of the immunocompetent cell having the anti-CD38 antibody on its cell surface of the present invention" is described. In the case where the immunocompetent cell to be infected with the anti-CD38 antigen-expressing retroviral virions is a cytotoxic T cell or natural killer cell, it is possible to produce a cytotoxic T cell or natural killer cell having the antibody expressed on its cell surface.
The resultant cytotoxic T cell or natural killer cell having the anti-CD38 antibody on its cell surface can destroy or kill specifically the above-mentioned target cells such as myeloma cells, malignant lymphoma cells, and leukemia cells by cytotoxic action.

In the case where the immunocompetent cell to be infected are cells such as macrophages and neutrophils, it is possible to produce cells having phagocytic capacity, such as macrophages and neutrophils having the anti-CD38 antibody on their cell surfaces. The resultant cells such as macrophages and neutrophils having the anti-CD38 antibody on their cell surfaces can destroy or kill specifically the above-mentioned target cells by the phagocytic activity or cytotoxic action.

Hereinafter, a process for purifying/isolating the immunocompetent cell having the anti-CD38 antibody on its cell surface and a process for evaluating or determining function of the immunocompetent cell are described.
The function of the immunocompetent cell having the anti-CD38 antibody on its cell surface to destroy or kill specifically the above-mentioned target cells can be evaluated or determined by an ordinary method such as flow cytometry based on the antibody staining method using the anti-CD38 antibody. Specifically, a time-dependent decrease in the number of target cells described above, which is caused by co-culture of the immunocompetent cell having the anti-CD38 antibody on its cell surface of the present invention with the above-mentioned target cells, is assayed or measured using a flow cytometer after an antigen-antibody reaction of the anti-CD38 antibody labeled with a fluorescent substance (such as phycoerythrin) and a non-cross reactive anti-CD38 antibody with the CD38 antigen present on the surfaces of the above-mentioned target cells.

The anti-CD38 antibody-expressing human T cell strain of the present invention has been deposited at the International Patent Organism Depositary of the National Institute of Advanced Industrial Science and Technology (Central 6, 1-1, Higashi 1-chome, Tsukuba-shi, Ibaraki-ken, 305-8566, Japan) on February 15, 2006 (FERM AP-20800).

The immunocompetent cell expressing the anti-CD38 antibody on its surface of the present invention has cytotoxicity against a cell having the CD38 antigen expressed at a high level on its cell surface and is useful for treatment of cancer. In addition, the method of producing the immunocompetent cell expressing the anti-CD38 antibody on its surface of the present invention enables sufficient immunocompetent cell production.
The cell capable of producing anti-CD38 antibody-expressing retroviral particles of the present invention (so-called packaging cell) is useful for producing retroviral particles capable of infecting any immunocompetent cells such as T cells, macrophages, natural killer cells, and neutrophils and releasing the viral virions (particles) extracellularly.

The antibody protein of the present invention can bind to a cell capable of expressing a large amount of the CD38 antigen with high affinity when expressed on the cell surface of the immunocompetent cell of the present invention to thereby be useful as a part of a protein forming the immunocompetent cell of the present invention. In addition, the DNA and retrovirus of the present invention are useful as template DNAs capable of expressing the anti-CD38 antibody.
The recombinant retroviral expression vector of the present invention is used for producing the packaging cell and is useful as a material for producing a recombinant cell.

The DNA coding anti-CD38 antibody of the present invention is useful as a material for producing the above-mentioned recombinant retroviral vector of the present invention.

### EXAMPLES

The present invention will be described in more detail based on the following examples, but the present invention is not limited thereto.

### [Cloning of anti-CD38 antibody gene]

First, total mRNA was extracted from 1 × 10⁶ THB7 cells (ATCC HB-136) as hybridoma cells using Trizol reagent (trade name, manufactured by GIBCO-BRL). Then, PCR was performed for 1 mg of the resultant total RNA to amplify (i) the H-chain variable region of the antibody (SEQ ID NO: 2) using the primers A_{H} to H_{H} described in a report by Ellis et al. (J. Immunology, 1995, 155: 925-937) and (ii) the L-chain variable region of the antibody (SEQ ID NO: 1) using the primers A_{L} to H_{L} described in the report, whereby 1 mg of cDNA coding the H-chain variable region (SEQ ID NO: 2) and 1 mg of cDNA coding the L-chain variable region (SEQ ID NO: 1) were produced, respectively.

From the resultant cDNAs coding the H-chain variable region (SEQ ID NO: 2) and the L-chain variable region (SEQ ID NO: 1), a DNA construct having an anti-CD38 antibody gene (cDNA) for expressing a complete anti-CD38 chimeric antibody was prepared according to a method described in Leukemia, 2004, 18: 676-684. Fig. 1 is a schematic view illustrating the sequence of the resultant DNA construct having the anti-CD38 antibody gene (cDNA).

### [Construction of anti-CD38 antibody expression retroviral vector]

The DNA construct having the anti-CD38 antibody gene was ligated to an *Eco*RI-*Xho*I restriction enzyme site of MSCV plasmid including genes of IRES and EGFP (hereinafter, referred to as "pMSCV-IRES-EGFP"). The pMSCV-IRES-EGFP was obtained from St. Jude Vector Development and Production Shared Resource (Memphis, TN, USA).

Subsequently, the presence of the L- and H-chain genes in the resultant anti-CD38 antibody expression retroviral vector was confirmed by a restriction enzyme treatment and electrophoresis.
Fig. 2 is an electrophoresis image. From this Fig. 2, it was confirmed that the anti-CD38 antibody expression retroviral vector included the L- and H-chain genes. The image shows a band of about 400 kbp of the L-chain DNA (lane 2) and a band of about 400 kbp of the H-chain DNA (lane 3) (lanes 1 and 4: molecular weight marker). The results reveal that the retroviral vector included the L- and H-chain genes.

### [Formation of anti-CD38 antibody-expressing retroviral particle]

The anti-CD38 antibody expression retroviral vector obtained above (1 mg) was transfected into packaging cells (number of cells: 1 x 10⁵, 293T: Riken Bioresource Center, Accession No. RCB2202) using 4 mg of Lipofection reagent (trade name, manufactured by GIBCO-BRL) by incubation in RPMI 1640 medium supplemented with fetal calf serum in a 6-well plate at 37°C for 3 hours, whereby a packaging cell capable of producing the anti-CD38 antibody-expressing retroviral particle (virions) were produced. The expression ratio was found to be 90% or more. Accession No.: FERM AP-20801. The resultant packaging cells capable of producing retroviral particles were incubated at 37°C for 24 to 96 hours, and the virus supernatant was collected (9 mL).

### [Production of T cell having anti-CD38 antibody on its cell surface of the present invention]

The resultant virus supernatant (3 mL, MOI 10⁵) and polybrene (final concentration 2 ng/ml: manufactured by SIGMA) were added to 10⁵ cells of a human T cell strain (Hut78 cell: from Institute of Development, Aging and Cancer, Tohoku University) in a 14-ml polypropylene tube (manufactured by Falcon), and the mixture was incubated at 37°C for 24 hours to infect the cells, whereby a human T cell having the anti-CD38 antibody on its cell surface (expression ratio: 90% or more) was prepared. The cell strain has been deposited before filing the present application (Accession No.: FERM AP-20800).

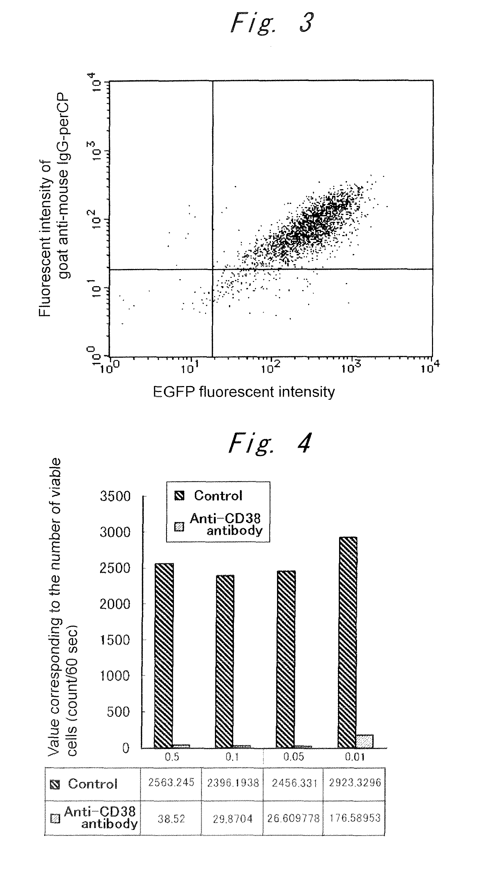

To detect expression of the antibody in the transgenic cells (EGFP positive cells), the expression pattern between EGFP and anti-mouse IgG-antibody-perCP was determined using an anti-mouse IgG antibody (trade name: FACSCalibur, manufactured by Becton, Dickinson and Company), and the results are shown as a two-dimensional plot (Fig. 3). The results suggest that the antibody was expressed on the cell surface of the transgenic cells (EGFP positive cells).

### [Test for evaluation of function of human T cell strain having anti-CD38 antibody on its cell surface]

The human T cells having the anti-CD38 antibody on their cell surfaces obtained above were co-cultured with myeloma cells derived from a patient at a ratio of 1:2 in a complete medium obtained by adding 10% fetal calf serum (manufactured by GIBCO-BRL) to RPMI-1640 medium in a 96-well plate (manufactured by Falcon) at 37°C for 2 to 5 days. Then, the myeloma cells were stained with 20 µl of non-cross reactive anti-CD38 antibody-phycoerythrin (manufactured by BD Biosciences), and viability of the myeloma cells was evaluated by flow cytometry. As a control, human T cells expressing no anti-CD38 antibody (from Institute of Development, Aging and Cancer, Tohoku University) were also co-cultured with the myeloma cells derived from the patient in the same way as above, and the cells were stained. Then, living cells were counted using the above-mentioned flow cytometer.

The evaluation test was performed by counting living myeloma cells remaining after co-culture of the myeloma cells with cells expressing the antibody or co-culture of the myeloma cells with cells expressing no antibody (control) for 5 days. The counting was carried out for a culture solution after 5 day-culture with flow cytometry to determine the number of cells which passed through a predetermined tube in the flow cytometry for 60 seconds. The counting was repeated five times under the same conditions, and the results were shown as mean values. When the number of myeloma cells co-cultured was defined as 1, in the case of the ratio of the human T cells expressing no anti-CD38 antibody to the myeloma cells is 0.5, 0.1, 0.05, or 0.01, the number of living myeloma cells was well over 2,000 (Fig. 4). On the other hand, when the number of co-cultured myeloma cells was defined as 1, in the case of the ratio of the human T cells having the anti-CD38 antibody on their cell surfaces to the myeloma cells was 0.5, 0.1, 0.05, or 0.01, almost all the myeloma cells were dead after 5 days.

### [Test of self-apoptosis suppression by anti-CD38 antibody]

Next, evaluation was performed on the self-apoptosis suppressing effect achieved by addition of another anti-CD38 antibody before expression of the anti-CD38 antibody by human immunocompetent cells infected with retroviral particles.
The evaluation process is as follows.
Human peripheral blood T cells were cultured in the presence of IL-2 and PHA for 48 hours, and a retrovirus was used to express the anti-CD38 antibody on the surfaces of the T cells. Before expression of the anti-CD38 antibody on the surfaces of the T cells, the anti-CD38 antibody was added at different concentrations. Then, on day 9, the survival rate was analyzed using a flow cytometer. A purified concentrate of the THB7 supernatant (antibody concentration 1.5 mg/ml) was diluted and used as coexistent anti-CD38 antibody.

Fig. 6 shows the results.
The results of Fig. 6 reveal that the effect of the coexistent anti-CD38 antibody was achieved in the cases where the concentration of the coexistent anti-CD38 antibody was 0.015 mg/ml or more with respect to 1 × 10⁵ to 1 × 10⁶ human T cells.
In particular, in the cases where the concentration of the coexistent anti-CD38 antibody was 0.15 mg/ml or more with respect to 1 × 10⁵ to 1 × 10⁶ human T cells, the self-apoptosis could be significantly prevented.

### (Test Example) [Evaluation of anti-tumor effect of anti-CD38 antibody-expressing cell]

Next, the anti-tumor effect of the cells expressing the anti-CD38 antibody was evaluated.
The evaluation method is as follows.
First, HT (malignant lymphoma)-Luc cells (1.6 × 10⁶) were transplanted into NOD/SCID mice. The term "Luc" refers to luciferase, which is used for non-disruptively measuring the size of a tumor using an image analyzer.
To the mice, transvenously injected were 0.8 × 10⁶ human T cells (separately transformed with a control vector or a vector including an anti-CD38 antibody gene) four times in total every 3 days after transplantation of HT-Luc. Rituximab was administered transperitoneally in an amount of 0.2 mg one day after the transplantation. Luciferin was administered to the mice on day 3 and 30, and the luminescence intensity was measured using a photon image analyzer.
The results are shown in the following table.

| Time course after transplantation | | Day 3 | Day 30 |
|---|---|---|---|
| Effector | | Ratio of mice having residual tumor * | |
| (A) Control | Intravenous injection | 6/6 (100%) | 6/6 (100%) |
| (B) Rituximab | Intraperitoneal injection | 5/6 (83.3%) | 2/6 (33.3%) |
| (C) T cell having anti-CD38 antibody | Intravenous injection | 5/6 (83.3%) | 1/6 (16.7%) |

| | | | |
|---|---|---|---|
| * By photon analyzer | | | |

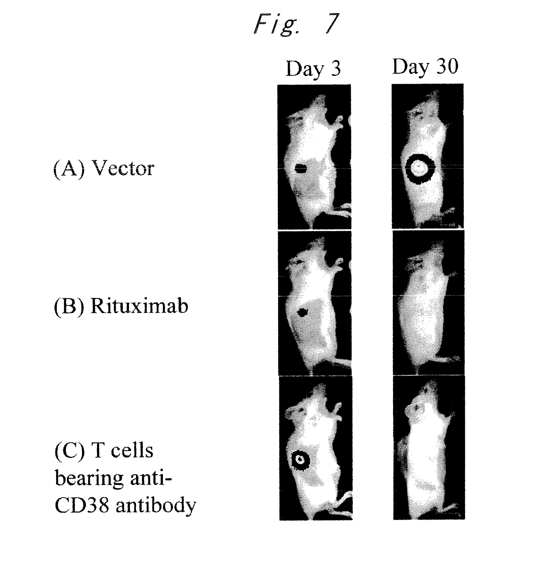

Fig. 7 shows pictures of mice taken by a photon image analyzer.
The results reveal that, in the case of transplantation of only the control vector, all the mice had residual tumors, while in the case of administration of the vector including a DNA coding the anti-CD38 antibody of the present invention, almost all the mice had no tumors. In addition, from the viewpoint of the luciferase activity and tumor size, the T cells having anti-CD38 antibody were found to achieve the effect equal to or higher than that of rituximab.

### INDUSTRIAL APPLICABILITY

The immunocompetent cell having the anti-CD38 antibody of the present invention has cytotoxicity against myeloma, malignant lymphoma, and cancer cells such as leukemia cells expressing CD38 antigens on their cell surfaces at a high level, and therefore, the cell is useful for treating the cancers. The treatment is unlike conventional chemotherapeutic agents and has no problem such as resistance of the chemotherapeutic agents. Moreover, the treatment is considered to cause no severe side-effects such as vomiting, diarrhea, and epilation, which may be caused by almost all chemotherapeutic agents.

Having described our invention as related to the present embodiments, it is our intention that the invention not be limited by any of the details of the description, unless otherwise specified, but rather be construed broadly within its spirit and scope as set out in the accompanying claims.

## Claims

1. An immunocompetent cell expressing an anti-CD38 antibody on its surface through genetic introduction of a DNA coding for part of the anti-CD38 antibody.

2. The immunocompetent cell according to Claim 1, wherein the anti-CD38 antibody is an antibody having the following amino acid sequence (a) or (b) in its variable region:
(a) An amino acid sequence set forth in SEQ ID NO: 1; and
(b) An amino acid sequence, in which one or more amino acids are being deleted, substituted or added in the amino acid sequence set forth in SEQ ID NO: 1.

3. The immunocompetent cell according to Claim 1 or 2, wherein the anti-CD38 antibody is an antibody that has the following amino acid sequence (c) or (d) in its heavy chain variable region:
(c) An amino acid sequence set forth in SEQ ID NO: 2; and
(d) An amino acid sequence, in which one or more amino acids are being deleted, substituted or added in the amino acid sequence set forth in SEQ ID NO: 2.

4. The immunocompetent cell according to Claim 1, wherein the DNA coding for part of the anti-CD38 antibody has a DNA coding the following amino acid sequence (b) and a DNA coding the following amino acid sequence (d) introduced thereinto:
(b) An amino acid sequence, in which one or more amino acids are being deleted, substituted or added in the amino acid sequence set forth in SEQ ID NO: 1; and
(d) An amino acid sequence, in which one or more amino acids are being deleted, substituted or added in the amino acid sequence set forth in SEQ ID NO: 2.

5. The immunocompetent cell according to Claim 1, wherein the anti-CD38 antibody has an amino acid sequence set forth in SEQ ID NO: 1 in part of its light chain and an amino acid sequence set forth in SEQ ID NO: 2 in part of its heavy chain.

6. The immunocompetent cell according to any one of Claims 1 to 5, wherein the immunocompetent cell is a T cell, natural killer cell, macrophage or polymorphonuclear leukocyte.

7. A protein of the following (a1), (b1), (c1) or (d1):
(a1) A protein having an amino acid sequence set forth in SEQ ID NO: 1;
(b1) A protein having an amino acid sequence, in which one or more amino acids are being deleted, substituted or added in the amino acid sequence set forth in SEQ ID NO: 1;
(c1) A protein having an amino acid sequence set forth in SEQ ID NO: 2; and
(d1) A protein having an amino acid sequence, in which one or more amino acids are being deleted, substituted or added in the amino acid sequence set forth in SEQ ID NO: 2.

8. A DNA coding the following amino acid sequence (a), (b), (c) or (d):
(a) An amino sequences set forth in SEQ ID NO: 1;
(b) An amino acid sequence, in which one or more amino acids are being deleted, substituted or added in the amino acid sequence set forth in SEQ ID NO: 1;
(c) An amino acid sequence set forth in SEQ ID NO: 2; and
(d) An amino acid sequence, in which one or more amino acids are being deleted, substituted or added in the amino acid sequence set forth in SEQ ID NO: 2.

9. A recombinant retrovirus having a DNA coding the following amino acid sequence (a), (b), (c) or (d):
(a) An amino acid sequence set forth in SEQ ID NO: 1;
(b) An amino acid sequence, in which one or more amino acids are being deleted, substituted or added in the amino acid sequence set forth in SEQ ID NO: 1;
(c) An amino acid sequence set forth in SEQ ID NO: 2; and
(d) An amino acid sequence, in which one or more amino acids are being deleted, substituted or added in the amino acid sequence set forth in SEQ ID NO: 2.

10. A packaging cell capable of producing particles of the recombinant retrovirus according to Claim 9.

11. An anti-CD38 antibody having the following amino acid sequence (a) or (b) in its light chain variable region and having the following amino acid sequence (c) or (d) in its heavy chain variable region:
(a) An amino acid sequence set forth in SEQ ID NO: 1;
(b) An amino acid sequence, in which one or more amino acids are being deleted, substituted or added in the amino acid sequence set forth in SEQ ID NO: 1;
(c) An amino acid sequence set forth in SEQ ID NO: 2; and
(d) An amino acid sequence, in which one or more amino acids are being deleted, substituted or added in the amino acid sequence set forth in SEQ ID NO: 2.

12. A method of producing an immunocompetent cell expressing an anti-CD38 antibody on its cell surface, comprising the steps of:
amplifying a cDNA of the anti-CD38 antibody using the mRNA coding part of the anti-CD38 antibody isolated from a hybridoma capable of producing the anti-CD38 antibody;
inserting the amplified cDNA into a retroviral vector;
transfecting the gene-inserted vector into a packaging cell to produce a packaging cell capable of producing an anti-CD38 antibody-expressing retroviral particle; and
infecting a human immunocompetent cell with the retroviral particle released from the packaging cell.

13. The method of producing an immunocompetent cell according to Claim 12, wherein the anti-CD38 antibody is the antibody according to Claim 11.

14. The method of producing an immunocompetent cell according to Claim 12 or 13, wherein the cDNA is the DNA according to Claim 8.

15. The method of producing an immunocompetent cell according to any one of Claims 12 to 14, wherein the packaging cell is the cell according to Claim 10.

16. The method of producing an immunocompetent cell according to any one of Claims 12 to 15, wherein the immunocompetent cell is the cell according to any one of Claims 1 to 6.

17. The method of producing an immunocompetent cell according to any one of Claims 12 to 16, wherein an anti-CD38 antibody is allowed to coexist when an anti-CD38 antibody is expressed from a human immunocompetent cell infected with the retroviral particle.


SEQUENCE LISTING

<110>  HIROSHIMA UNIVERSITY
       MIHARA, Keichiro

<120>  Immunocompetent cell having anti-CD38 antibodies thereon

<130>  P67122PCT

<150>  JP 2006-156595
<151>  2006-06-05

<160>  2

<170>  PatentIn version 3.3

<210>  1
<211>  120
<212>  PRT
<213>  THB7 cell

<400>  1

Asp Ile Gln Leu Thr Gln Ser Ser Ser Ser Phe Ser Val Ser Leu Gly
1               5                   10                  15

Asp Arg Val Thr Ile Thr Cys Lys Ala Ser Glu Asp Ile Tyr Asn Arg
            20                  25                  30

Leu Ala Trp Tyr Gln Gln Lys Pro Gly Asn Ala Pro Arg Leu Leu Ile
        35                  40                  45

Ser Gly Ala Thr Ser Leu Glu Thr Gly Val Pro Ser Arg Phe Ser Gly
    50                  55                  60

Ser Gly Ser Gly Lys Asp Tyr Thr Leu Ser Ile Thr Ser Leu Gln Thr
65                  70                  75                  80

Glu Asp Val Ala Thr Tyr Tyr Cys Gln Gln Tyr Trp Ser Thr Pro Thr
                85                  90                  95

Phe Gly Gly Gly Thr Lys Leu Glu Ile Lys Arg Ala Asp Ala Ala Pro
            100                 105                 110

Thr Val Ser Ile Phe Pro Pro Ser
        115                 120


<210>  2
<211>  119
<212>  PRT
<213>  THB7 cell

<400>  2

Gln Val Gln Leu Lys Gln Ser Gly Pro Ser Leu Val Gln Pro Ser Gln
1               5                   10                  15

Arg Leu Ser Ile Thr Cys Thr Val Ser Gly Phe Ser Leu Ile Ser Tyr
            20                  25                  30

Gly Val His Trp Val Arg Gln Ser Pro Gly Lys Gly Leu Glu Trp Leu
        35                  40                  45

Gly Val Ile Trp Arg Gly Gly Ser Thr Asp Tyr Asn Ala Ala Phe Met

50                  55                  60

Ser Arg Leu Ser Ile Thr Lys Asp Asn Ser Lys Ser Gln Val Phe Phe
65                  70                  75                  80

Lys Met Asn Ser Leu Gln Ala Asp Asp Thr Ala Ile Tyr Phe Cys Ala
                85                  90                  95

Lys Thr Leu Ile Thr Thr Gly Tyr Ala Met Asp Tyr Trp Gly Gln Gly
            100                 105                 110

Thr Ser Val Thr Val Ser Ser
        115